# EUROPÄISCHE PATENTSCHRIFT

(11) **EP 2 083 893 B1**
(45) Veröffentlichungstag und Bekanntmachung des Hinweises auf die Patenterteilung: **09.12.2009**
(21) Anmeldenummer: 07803274.5
(22) Anmeldetag: 06.09.2007
(51) Int. Cl.: A61M 5/32, B65B 7/28

(54) **VORRICHTUNG UND VERFAHREN ZUR MONTAGE EINES NADELSCHUTZES AUF EINEN SPRITZENKÖRPER**
DEVICE AND METHOD FOR MOUNTING A NEEDLE GUARD ONTO A SYRINGE BODY
DISPOSITIF ET PROCÉDÉ POUR LE MONTAGE D'UNE PROTECTION D'AIGUILLE SUR UN CORPS DE SERINGUE

(30) Priorität: 28.09.2006 DE 102006045926; 20.10.2006 DE 102006049528
(43) Veröffentlichungstag der Anmeldung: 05.08.2009
(73) Patentinhaber: Robert Bosch GmbH, 70442 Stuttgart (DE)
(72) Erfinder: LEIDIG, Juergen, 74586 Frankenhardt (DE)
(86) Internationale Anmeldenummer: PCT/EP2007/059314
(87) Internationale Veröffentlichungsnummer: WO 2008/037575

(56) Entgegenhaltungen:
- WO-A-90/00414
- US-A- 5 024 666
- US-A- 5 078 696
- US-A- 5 469 964

## Beschreibung

### Stand der Technik

Die vorliegende Erfindung betrifft eine Vorrichtung zur Montage eines Nadelschutzes auf einen Spritzenkörper sowie ein Verfahren zur Montage eines Nadelschutzes auf eine Spritze.

Aus dem Stand der Technik sind unterschiedliche Vorrichtungen zum Zuführen von Verschlüssen, insbesondere für pharmazeutische Behältnisse, bekannt. Derartige Vorrichtungen umfassen beispielsweise einen Vibrationsfördertopf, welcher die Verschlüsse orientiert und in eine Förderrinne abgibt. Die Verschlüsse werden einem Verschließrad zugeführt, welches die einzelnen Verschlüsse der Förderrinne entnimmt und bei seiner weiteren Drehung auf Flaschen aufsetzt und anschließend verschließt.

Eine derartige Vorrichtung ist beispielsweise aus der DE 103 22 476 A1 bekannt. Hierbei sind eine Sortiereinrichtung und die Förderrinne miteinander gekoppelt, welche als Einheit gemeinsam verfahrbar ist. Insbesondere bei einem Verschließen von einer mit einer Nadel versehenen Spritze mit einem Nadelschutz kann im Stand der Technik jedoch öfters ein Zustand vorkommen, in welchem der Nadelschutz schräg aufgesetzt wird, so dass die Nadel der Spritze den Nadelschutz berührt und eventuell verbogen wird, oder die Nadel den Nadelschutz durchsticht. Ferner kann es vorkommen, dass der Verschluss nicht mit der notwendigen Aufsetzkraft auf die Spritze aufgesetzt wird.

### Vorteile der Erfindung

Die erfindungsgemäße Vorrichtung zur Montage eines Nadelschutzes auf einen Spritzenkörper mit den Merkmalen des Patentanspruchs 1 weist demgegenüber den Vorteil auf, dass der Nadelschutz schnell und sicher auf den Spritzenkörper aufgesetzt werden kann, ohne dass dabei ein Kontakt zwischen dem Nadelschutz und einer Nadel des Spritzenkörpers auftritt. Hierdurch ist es möglich, insbesondere Spritzenkörper mit aufgeklebten Nadeln, welche besonders empfindlich auf Berührungen durch den Nadelschutz sind, zu verwenden. Die erfindungsgemäße Vorrichtung umfasst dabei eine Halteeinrichtung zum Halten des Spritzenkörpers, eine Nadelschutzaufnahme zum Aufnehmen des Nadelschutzes und eine Drucklufteinrichtung zur Bereitstellung von Druckluft. Die Vorrichtung umfasst ferner eine erste Bewegungseinrichtung zur Bewegung der Nadelschutzaufnahme in Vertikalrichtung und eine Rotationseinrichtung, um die Nadelschutzaufnahme während der Vertikalbewegung zumindest teilweise zu drehen. Die Nadelschutzaufnahme ist dabei unterhalb der Halteeinrichtung angeordnet, so dass das Aufsetzen des Nadelschutzes auf den Spritzenkörper von unten erfolgt. Die Druckluft wird der Nadelschutzaufnahme derart zugeführt, dass der Nadelschutz auf einem Luftpolster in der Nadelschutzaufnahme schwebt und rotiert und somit einfach zentrisch auf den Spritzenkörper aufgesetzt werden kann.

Die Unteransprüche zeigen bevorzugte Weiterbildungen der Erfindung.

Vorzugsweise umfasst die Vorrichtung ferner einen Niederhalter, welcher oberhalb der Halteeinrichtung des Spritzenkörpers angeordnet ist. Der Niederhalter ist mit dem anderen Ende des Spritzenkörpers in Kontakt bringbar und stellt eine Gegenkraft gegen die Aufsetzkraft zum Aufsetzen des Nadelschutzes auf den Spritzenkörper bereit.

Der Niederhalter ist vorzugsweise in Vertikalrichtung mittels einer zweiten Bewegungseinrichtung bewegbar. Dadurch ist es möglich, vorzugsweise den Niederhalter und die Nadelschutzaufnahme gleichzeitig in entgegengesetzte Richtungen zu bewegen, um den Nadelschutz auf den Spritzenkörper aufzusetzen.

Weiter bevorzugt umfasst die erfindungsgemäße Vorrichtung einen Kraftsensor, um eine Kraft des Aufsetzens des Nadelschutzes auf den Spritzenkörper aufzunehmen. Die Verwendung des Kraftsensors ermöglicht es, dass anhand der Aufsetzkraft bestimmt werden kann, ob der Nadelschutz richtig und sicher auf den Spritzenkörper aufgesetzt wurde. Dadurch können schon beim Aufsetzen des Nadelschutzes Spritzen mit einem mit zu großer oder zu kleiner Aufsetzkraft aufgesetzten Nadelschutz aussortiert werden.

Der Kraftsensor ist vorzugsweise am Niederhalter angeordnet. Dadurch kann ein besonders kompakter Aufbau sichergestellt werden.

Ferner umfasst die erfindungsgemäße Vorrichtung vorzugsweise eine Steuereinrichtung mit einem Speicher, wobei in dem Speicher wenigstens ein vorbestimmter Kraftwert für die Aufsetzkraft gespeichert ist. Die Steuereinrichtung dient dazu, eine aufgenommene Aufsetzkraft mit der vorbestimmten Aufsetzkraft zu vergleichen und gegebenenfalls bei falscher Aufsetzkraft die Spritze auszusortieren. Es sei angemerkt, dass hierbei sowohl eine untere Grenze für die Aufsetzkraft als auch eine obere Grenze für die Aufsetzkraft überprüft werden kann.

Besonders bevorzugt werden die erste Bewegungseinrichtung und die zweite Bewegungseinrichtung gleichzeitig aktiviert. Dadurch kann eine besonders kurze Zeit zum Aufsetzen des Nadelschutzes auf den Spritzenkörper sichergestellt werden.

Die Erfindung betrifft ferner ein Verfahren zur Montage eines Nadelschutzes auf einen Spritzenkörper, welches die Schritte des Haltens des Spritzenkörpers mittels einer Halteeinrichtung und des Einlegens des Nadelschutzes in eine Nadelschutzaufnahme umfasst. Die Nadelschutzaufnahme ist dabei unterhalb des Spritzenkörpers angeordnet. Ferner wird Druckluft bereitgestellt, welche zur Nadelschutzaufnahme zugeführt wird, um den Nadelschutz in der Nadelschutzaufnahme in einen auf einem Luftkissen schwebenden Zustand zu versetzen. Wenn sich der Nadelschutz in dem schwebenden Zustand befindet, wird das Aufsetzen des Nadelschutzes auf den Spritzenkörper durch vertikales Bewegen der Nadelschutzaufnahme in Richtung zum Spritzenkörper bewirkt, wobei während des Aufsetzvorgangs die Nadelschutzaufnahme eine Drehbewegung ausführt. Dadurch wird ein sicheres Zentrieren der Nadelschutzaufnahme auf dem Spritzenkörper erreicht.

Das Verfahren umfasst weiter bevorzugt das Aufnehmen einer Aufsetzkraft des Nadelschutzes auf den Spritzenkörper, insbesondere um Spritzen mit falsch aufgesetztem Nadelschutz auszusortieren. Eine Spritze wird insbesondere dann aussortiert, wenn die Aufsetzkraft kleiner oder größer als ein vorbestimmter Wert ist.

### Zeichnung

Nachfolgend wird ein bevorzugtes Ausführungsbeispiel der Erfindung unter Bezugnahme auf die begleitende Zeichnung im Detail beschrieben. In der Zeichnung ist:
- Figur 1: eine schematische Schnittansicht einer Vorrichtung zur Montage eines Nadelschutzes auf einen Spritzenkörper,
- Figur 2: eine vergrößerte, teilweise geschnittene Ansicht der Vorrichtung von Figur 1 während des Aufsetzvorgangs,
- Figur 3: eine schematische, teilweise geschnittene Ansicht der Vorrichtung, nachdem der Nadelschutz aufgesetzt wurde und
- Figur 4: eine schematische, teilweise geschnittene Ansicht der Vorrichtung, welche in ihren Ausgangszustand zurückgefahren wird.

### Bevorzugte Ausführungsformen der Erfindung

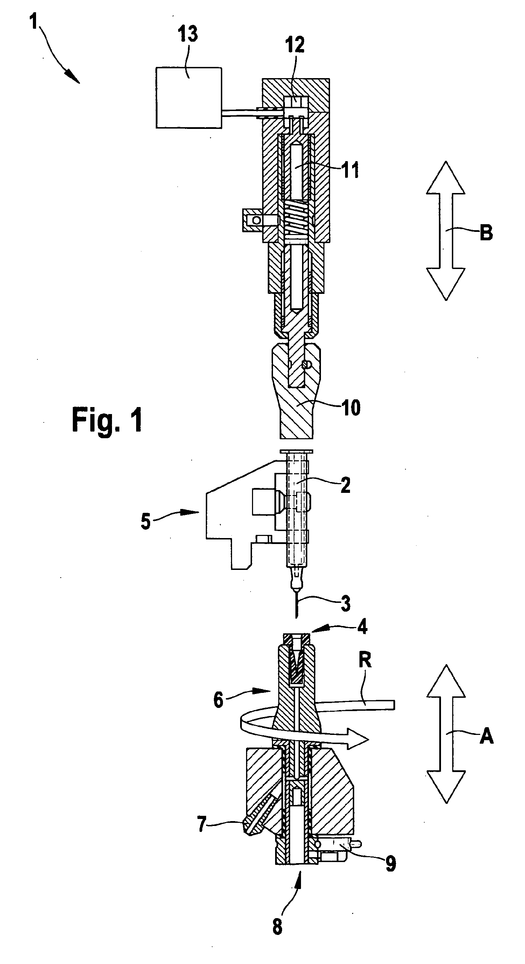

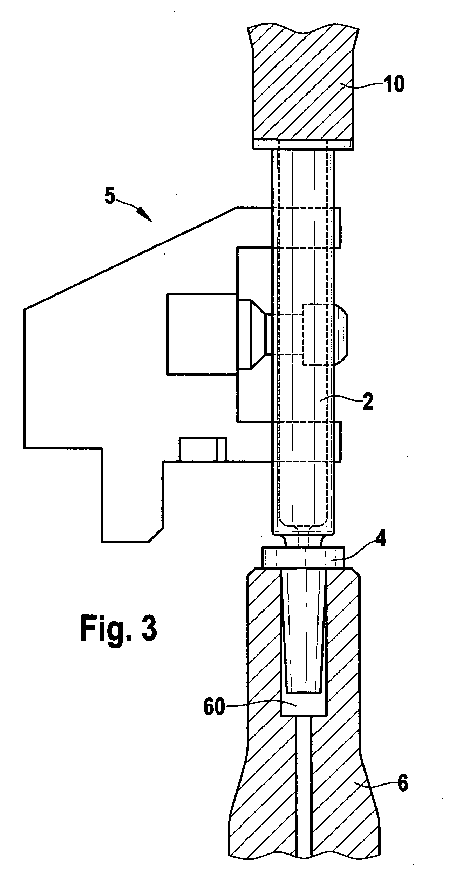

Nachfolgend wird unter Bezugnahme auf die Figuren 1 bis 4 eine Vorrichtung 1 zur Montage eines Nadelschutzes 4 auf einen Spritzenkörper 2 im Detail beschrieben.

Figur 1 zeigt schematisch den Gesamtaufbau der Vorrichtung 1. Die Vorrichtung 1 umfasst eine Halteeinrichtung 5 zum Halten des Spritzenkörpers 2. Die Halteeinrichtung 5 kann beispielsweise eine Greifeinheit o. Ä. sein. Die Vorrichtung 1 umfasst ferner eine Nadelschutzaufnahme 6 und einen Niederhalter 10. Wie in Figur 1 durch den Doppelpfeil A angedeutet, kann die Nadelschutzaufnahme 6 in Vertikalrichtung hoch und runter in Richtung der Halteeinrichtung 5 bewegt werden. Ferner kann die Nadelschutzaufnahme in Richtung des Pfeils R rotiert werden. Hierbei wird die gesamte Nadelschutzaufnahme 6 rotiert bzw. in Vertikalrichtung bewegt. Die Rotation wird dabei über einen vorbestimmten Winkel, z.B. 90°, durchgeführt. Die Nadelschutzaufnahme 6 wird mittels einer ersten Bewegungseinrichtung 8 in Vertikalrichtung bewegt. Die erste Bewegungseinrichtung 8 ist beispielsweise ein Druckmittel-betätigter Kolben. Die Rotation in Richtung des Pfeils R kann mittels einer Rotationseinrichtung 9 bereitgestellt werden. Der Niederhalter 10 kann, wie durch den Doppelpfeil B angedeutet, ebenfalls in Vertikalrichtung hoch und runter bewegt werden. Die Bewegung des Niederhalters 10 erfolgt mittels einer zweiten Bewegungseinrichtung 11, welche beispielsweise ein federbelasteter Kolben o. Ä. ist. Der Kolben kann druckmittelbetätigt sein und nach Wegnahme des Druckmittels erfolgt eine automatische Rückstellung durch eine vorgespannte Feder.

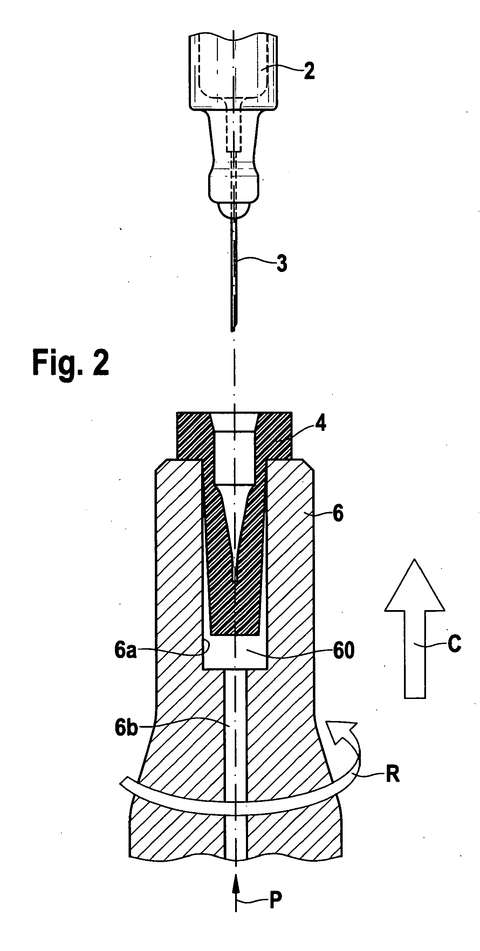

Die Vorrichtung 1 umfasst ferner eine Drucklufteinrichtung 7, um Druckluft an der Nadelschutzaufnahme 6 bereitzustellen. Die Nadelschutzaufnahme 6 umfasst eine im Wesentlichen becherförmige Aufnahme 6a, welche an ihrem Boden eine Öffnung aufweist, die mit einem Druckluftzufuhrkanal 6b verbunden ist. Dies ist in Figur 2 erkennbar. Druckluft wird, wie durch den Pfeil P in Figur 2 angedeutet, durch den Druckluftzufuhrkanal 6b in die becherförmige Aufnahme 6a zugeführt. Dadurch entsteht in der becherförmigen Aufnahme 6a ein Luftpolster 60, auf welchem der Nadelschutz 4 schwebend gehalten ist.

Am Niederhalter 10 ist ferner ein Kraftmesssensor 12 angeordnet. Der Kraftmesssensor 12 dient zur Aufnahme einer Aufsetzkraft, mit welcher der Nadelschutz 4 auf den Spritzenkörper 2 aufgesetzt wird. Der Kraftsensor 12 ist mit einer Steuereinrichtung 13 verbunden. Die Steuereinrichtung 13 umfasst einen Speicher, in welchem vorbestimmte obere und untere Grenzwerte für die Aufsetzkraft abgespeichert sind. Die Steuereinrichtung 13 vergleicht dabei den aktuellen Wert der Aufsetzkraft mit den abgespeicherten Werten und gibt gegebenenfalls bei einer Abweichung ein entsprechendes Signal aus und sortiert die mit einer falschen Aufsetzkraft aufgesetzte Spritze aus.

Die Funktion der erfindungsgemäßen Vorrichtung 1 ist dabei wie folgt: In einem ersten Schritt wird ein Spritzenkörper 2, auf welchen eine Nadel 3 aufgesetzt wurde, durch die Halteeinrichtung 5 gehalten. Die Nadel 3 kann beispielsweise mittels Kleben auf dem Spritzenkörper 2 befestigt werden. Gleichzeitig oder anschließend wird ein Nadelschutz 4 in die Nadelschutzaufnahme 6, genauer in die becherförmige Aufnahme 6a, angeordnet. Dies kann z. B. mittels einer Förderrinne erfolgen. Die Nadelschutzaufnahme 6 und der Niederhalter 10 befinden sich in ihren Ausgangspositionen. Diese Situation ist in Figur 1 schematisch dargestellt. Wenn nun der Nadelschutz 4 auf den Spritzenkörper 2 aufgesetzt werden soll, wird zuerst Druckluft mittels der Drucklufteinrichtung 7 erzeugt bzw. aus einem Druckluftspeicher entnommen und über den Druckluftzufuhrkanal 6b in den unteren Bereich der becherförmigen Aufnahme 6a zugeführt. Dadurch entsteht ein Luftpolster 60, auf welchem der Nadelschutz 4 schwebt. Dieser Zustand ist in Figur 2 dargestellt. In einem nächsten Schritt wird die Nadelschutzaufnahme 6 in Vertikalrichtung nach oben in Richtung auf den Spritzenkörper 2 zu bewegt. Dies ist durch den Pfeil C in Figur 2 angedeutet. Gleichzeitig erfolgt eine Rotation der Nadelschutzaufnahme 6 um einen vorbestimmten Winkel, z.B. 90°. Die Rotation erfolgt somit gleichzeitig mit der Vertikalbewegung der Nadelschutzaufnahme 6.

Während sich die Nadelschutzaufnahme 6 nach oben in Richtung des Spritzenkörpers 2 bewegt, bewegt sich gleichzeitig der Niederhalter 10 in Vertikalrichtung nach unten. Der Niederhalter 10 wird dabei so lange bewegt, bis er mit der Rückseite des Spritzenkörpers 2 in Kontakt kommt, um einen Widerstand beim Aufsetzen des Nadelschutzes 4 auf den Spritzenkörper 2 bereitzustellen. Der Niederhalter 10 muss dabei vor dem eigentlichen Aufsetzvorgang des Nadelschutzes 4 am entgegengesetzten Ende des Spritzenkörpers 2 anliegen, um den Aufsetzwiderstand bereitzustellen. Die Nadelschutzaufnahme 6 wird weiter in Richtung des Spritzenkörpers 2 bewegt, bis sich der Nadelschutz 4 über die Nadel 3 auf einen vorzugsweise sich verjüngenden Bereich des Spritzenkörpers 2 anlegt. Durch das Luftpolster 60 und die Rotationsbewegung R wird der Nadelschutz 4 dabei zentriert. Das Aufsetzen des Nadelschutzes 4 erfolgt somit mit einer Aufsetzkraft, welche durch den Kraftmesssensor 12 am anderen Ende des Niederhalters 10 aufgenommen wird. Der Kraftmesssensor 12 gibt die aufgenommene Aufsetzkraft an die Steuereinrichtung 13 weiter, welche die aktuelle Aufsetzkraft mit einer abgespeicherten Aufsetzkraft vergleicht. Im Falle von einer vorbestimmten Abweichung gibt die Steuereinrichtung 13 ein entsprechendes Signal aus und der Spritzenkörper mit dem falsch aufgesetzten Nadelschutz wird aussortiert.

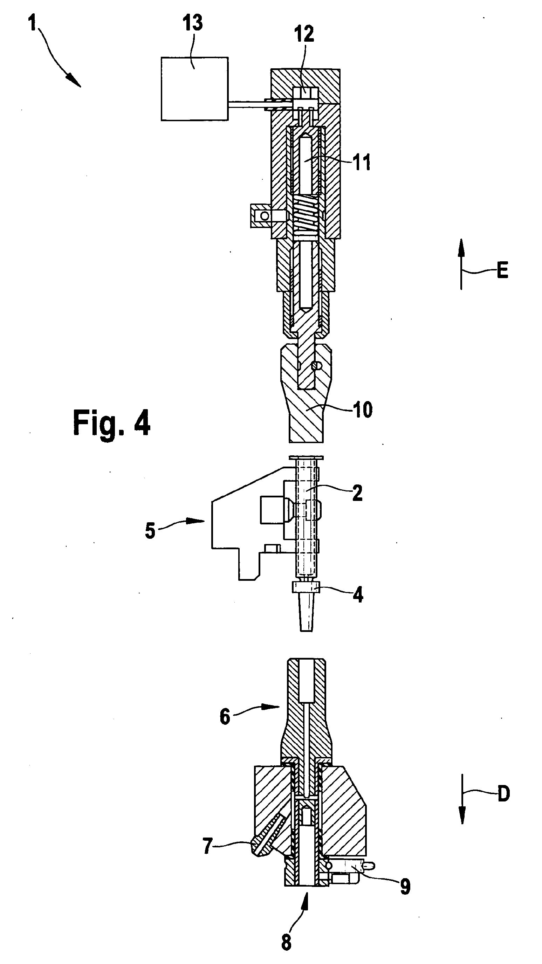

Wenn der Nadelschutz 4 auf den Spritzenkörper 2 aufgesetzt ist, werden die Nadelschutzaufnahme 6 und der Niederhalter 10 wieder gleichzeitig in entgegengesetzten Richtungen bewegt, d.h. die Nadelschutzaufnahme 6 wird nach unten bewegt, wie in Figur 4 durch den Pfeil D angedeutet, und der Niederhalter 10 wird nach oben bewegt, wie in Figur 4 durch den Pfeil E angedeutet.

Damit ist das Aufsetzen des Nadelschutzes 4 auf den Spritzenkörper 2 abgeschlossen und die Halteeinrichtung 5 kann den mit Nadelschutz versehenen Spritzenkörper abtransportieren oder an eine entsprechende Transporteinrichtung übergeben.

Erfindungsgemäß wird somit der Nadelschutz 4 von unten auf den Spritzenkörper 2 aufgesetzt, wobei der Nadelschutz 4 auf einem Luftpolster 60 schwebt. Dadurch wird eine sichere Zentrierung des Nadelschutzes 4 während des Aufsetzvorgangs sichergestellt. Dies wird zusätzlich noch durch die Rotation der Nadelschutzaufnahme 6 während des Aufsetzens unterstützt. Die Aufsetzkraft wird mittels des Kraftmesssensors 12 überwacht, so dass die Vorrichtung 1 sofort falsch aufgesetzte Spritzen aussortieren kann. Dadurch kann insbesondere vermieden werden, dass Spritzen mit falsch oder schlecht aufgesetztem Nadelschutz vertrieben werden.

## Patentansprüche

1. Vorrichtung zur Montage eines Nadelschutzes (4) auf einen Spritzenkörper (2), welcher mit einer Nadel (3) versehen ist, umfassend eine Halteeinrichtung (5) zum Halten des Spritzenkörpers (2), eine Nadelschutzaufnahme (6) zum Aufnehmen des Nadelschutzes (4), eine Drucklufteinrichtung (7) zum Bereitstellen von Druckluft, eine erste Bewegungseinrichtung (8) zum Bewegen der Nadelschutzaufnahme (6) in Vertikalrichtung (A) und eine Rotationseinrichtung (9), um die Nadelschutzaufnahme (6) zumindest teilweise zu drehen, wobei die Nadelschutzaufnahme (6) unterhalb der Halteeinrichtung (5) angeordnet ist, wobei die Druckluft der Nadelschutzaufnahme (6) derart zugeführt wird, dass der Nadelschutz (4) auf einem Luftpolster (60) schwebend gehalten ist, und wobei die Nadelschutzaufnahme (6) mittels der ersten Bewegungseinrichtung (8) in Vertikalrichtung (A) bewegbar ist.

2. Vorrichtung nach Anspruch 1, ferner umfassend einen Niederhalter (10), welcher oberhalb der Halteeinrichtung (5) angeordnet ist und mit einem dem Nadelschutz abgewandten Ende des Spritzenkörpers (2) in Kontakt bringbar ist.

3. Vorrichtung nach Anspruch 2, **dadurch gekennzeichnet, dass** der Niederhalter (10) in Vertikalrichtung mittels einer zweiten Bewegungseinrichtung (11) bewegbar ist.

4. Vorrichtung nach einem der vorhergehenden Ansprüche, ferner umfassend einen Kraftmesssensor (12), um eine Aufsetzkraft während des Aufsetzens des Nadelschutzes (4) auf den Spritzenkörper (2) aufzunehmen.

5. Vorrichtung nach Anspruch 4, **dadurch gekennzeichnet, dass** der Kraftmesssensor (12) am Niederhalter (10) angeordnet ist.

6. Vorrichtung nach Anspruch 4 oder 5, ferner umfassend eine Steuereinrichtung (13) mit einem Speicher, wobei im Speicher wenigstens ein vorbestimmter Wert für die Aufsetzkraft gespeichert ist, und die Steuereinrichtung einen aufgenommenen Wert der Aufsetzkraft mit dem vorbestimmten Wert der Aufsetzkraft vergleicht.

7. Vorrichtung nach einem der Ansprüche 3 bis 6, **dadurch gekennzeichnet, dass** die erste Bewegungseinrichtung (8) und die zweite Bewegungseinrichtung (11) gleichzeitig aktivierbar sind.

8. Verfahren zur Montage eines Nadelschutzes (4) auf einen Spritzenkörper (2), umfassend die Schritte:
- Halten des Spritzenkörpers (2) mittels einer Halteeinrichtung (5),
- Einlegen des Nadelschutzes (4) in eine Nadelschutzaufnahme (6), wobei die Nadelschutzaufnahme (6) unterhalb des Spritzenkörpers (2) angeordnet ist,
- Bereitstellen von Druckluft in der Nadelschutzaufnahme (6), um den Nadelschutz (4) in der Nadelschutzaufnahme (6) auf einem Luftpolster (60) in einen schwebenden Zustand zu versetzen,
- Bewegen der Nadelschutzaufnahme (6) in Richtung des Spritzenkörpers (2), um den Nadelschutz (4) auf den Spritzenkörper (2) aufzusetzen, wobei gleichzeitig die Nadelschutzaufnahme (6) eine Rotationsbewegung durchführt.

9. Verfahren nach Anspruch 8, **dadurch gekennzeichnet, dass** eine Aufsetzkraft während des Aufsetzens des Nadelschutzes (4) auf den Spritzenkörper (2) mittels eines Kraftsensors (12) aufgenommen wird.

10. Verfahren nach Anspruch 9, **dadurch gekennzeichnet, dass** wenn die Aufsetzkraft eine vorbestimmte Abweichung von einer vorbestimmten Aufsetzkraft aufweist, der Spritzenkörper (2) aussortiert wird.

## Claims

1. Device for mounting a needle guard (4) onto a syringe body (2) which is provided with a needle (3), the device comprising a retaining device (5) for retaining the syringe body (2), a needle guard receptacle (6) for receiving the needle guard (4), a compressed air device (7) for providing compressed air, a first movement device (8) for moving the needle guard receptacle (6) in a vertical direction (A), and a rotation device (9) for at least partially rotating the needle guard receptacle (6), wherein the needle guard receptacle (6) is arranged below the retaining device (5), wherein the compressed air is supplied to the needle guard receptacle (6) in such a manner that the needle guard (4) is suspended on an air cushion (60), and wherein the needle guard receptacle (6) is movable in a vertical direction (A) by means of the first movement device (8).

2. Device according to Claim 1, furthermore comprising a holding-down device (10) which is arranged above the retaining device (5) and can be brought into contact with an end of the syringe body (2), which end faces away from the needle guard.

3. Device according to Claim 2, **characterized in that** the holding-down device (10) is movable in a vertical direction by means of a second movement device (11).

4. Device according to one of the preceding claims, furthermore comprising a force-measuring sensor (12) in order to record an attachment force during the attaching of the needle guard (4) to the syringe body (2).

5. Device according to Claim 4, **characterized in that** the force-measuring sensor (12) is arranged on the holding-down device (10).

6. Device according to Claim 4 or 5, furthermore comprising a control device (13) with a memory, wherein at least one predetermined value for the attachment force is stored in the memory, and the control device compares a recorded value for the attachment force with the predetermined value for the attachment force.

7. Device according to one of Claims 3 to 6, **characterized in that** the first movement device (8) and the second movement device (11) can be activated simultaneously.

8. Method for mounting a needle guard (4) onto a syringe body (2), comprising the following steps:
- retaining the syringe body (2) by means of a retaining device (5),
- inserting the needle guard (4) into a needle guard receptacle (6), wherein the needle guard receptacle (6) is arranged below the syringe body (2),
- providing compressed air in the needle guard receptacle (6) in order to place the needle guard (4) in the needle guard receptacle (6) into a suspended state on an air cushion (60),
- moving the needle guard receptacle (6) in the direction of the syringe body (2) in order to attach the needle guard (4) to the syringe body (2), wherein the needle guard receptacle (6) carries out a rotational movement at the same time.

9. Method according to Claim 8, **characterized in that** an attachment force during the attaching of the needle guard (4) to the syringe body (2) is recorded by means of a force sensor (12).

10. Method according to Claim 9, **characterized in that** if the attachment force has a predetermined deviation from a predetermined attachment force, the syringe body (2) is discarded.

## Revendications

1. Dispositif pour le montage d'une protection d'aiguille (4) sur un corps de seringue (2), qui est pourvu d'une aiguille (3), comprenant un dispositif de retenue (5) pour retenir le corps de seringue (2), un logement de protection d'aiguille (6) pour recevoir la protection d'aiguille (4), un dispositif à air comprimé (7) pour fournir de l'air comprimé, un premier dispositif de déplacement (8) pour déplacer le logement de protection d'aiguille (6) dans la direction verticale (A) et un dispositif de rotation (9) pour faire tourner au moins en partie le logement de protection d'aiguille (6), le logement de protection d'aiguille (6) étant disposé sous le dispositif de retenue (5), l'air comprimé étant acheminé au logement de protection d'aiguille (6) de telle sorte que la protection d'aiguille (4) soit retenue en suspension sur un coussin d'air (60), le logement de protection d'aiguille (6) pouvant être déplacé au moyen du premier dispositif de déplacement (8) dans la direction verticale (A).

2. Dispositif selon la revendication 1, comprenant en outre un dispositif de maintien vers le bas (10) qui est disposé au-dessus du dispositif de retenue (5), et qui peut être amené en contact avec une extrémité du corps de seringue (2) opposée à la protection d'aiguille.

3. Dispositif selon la revendication 2, **caractérisé en ce que** le dispositif de maintien vers le bas (10) peut être déplacé dans la direction verticale au moyen d'un deuxième dispositif de déplacement (11) .

4. Dispositif selon l'une quelconque des revendications précédentes, comprenant en outre un capteur de mesure de force (12) afin d'enregistrer une force d'application pendant l'application de la protection d'aiguille (4) sur le corps de seringue (2).

5. Dispositif selon la revendication 4, **caractérisé en ce que** le capteur de mesure de force (12) est disposé sur le dispositif de maintien vers le bas (10).

6. Dispositif selon la revendication 4 ou 5, comprenant en outre un dispositif de commande (13) avec une mémoire, au moins une valeur prédéterminée pour la force d'application étant mémorisée dans la mémoire, et le dispositif de commande comparant une valeur reçue de la force d'application avec la valeur prédéterminée de la force d'application.

7. Dispositif selon l'une quelconque des revendications 3 à 6, **caractérisé en ce que** le premier dispositif de déplacement (8) et le deuxième dispositif de déplacement (11) peuvent être activés en même temps.

8. Procédé de montage d'une protection d'aiguille (4) sur un corps de seringue (2), comprenant les étapes suivantes :
- maintien du corps de seringue (2) au moyen d'un dispositif de retenue (5),
- insertion de la protection d'aiguille (4) dans un logement de protection d'aiguille (6), le logement de protection d'aiguille (6) étant disposé sous le corps de seringue (2),
- fourniture d'air comprimé dans le logement de protection d'aiguille (6), afin d'amener la protection d'aiguille (4) dans le logement de protection d'aiguille (6) dans un état de suspension sur un coussin d'air (60),
- déplacement du logement de protection d'aiguille (6) dans la direction du corps de seringue (2), afin d'appliquer la protection d'aiguille (4) sur le corps de seringue (2), le logement de protection d'aiguille (6) effectuant simultanément un mouvement de rotation.

9. Procédé selon la revendication 8, **caractérisé en ce qu'**une force d'application est enregistrée au moyen d'un capteur de force (12) pendant l'application de la protection d'aiguille (4) sur le corps de seringue (2).

10. Procédé selon la revendication 9, **caractérisé en ce que**, lorsque la force d'application présente un écart prédéterminé par rapport à une force d'application prédéterminée, le corps de seringue (2) est extrait.
